# FASCICULE DE BREVET EUROPEEN

(11) **EP 0 453 467 B1**
(45) Date de publication et mention de la délivrance du brevet: **21.07.1993**
(21) Numéro de dépôt: 90901644.6
(22) Date de dépôt: 05.01.1990
(51) Int. Cl.: A61M 5/50

(54) **SERINGUES JETABLES NON REUTILISABLES ET PROCEDES DE FABRICATION**
WEGWERFEINSPRITZNADEL FÜR EINMALIGE VERWENDUNG SOWIE VERFAHREN ZUR HERSTELLUNG
DISPOSABLE SYRINGE FOR ONCE-ONLY USE AND MANUFACTURING PROCESSES

(30) Priorité: 10.01.1989 FR 8900345
(43) Date de publication de la demande: 30.10.1991
(73) Titulaire: FENET, Emeric Guy, F-13122 Ventabren (FR)
(72) Inventeur: FENET, Emeric Guy, F-13122 Ventabren (FR)
(74) Mandataire: Moretti, René
(86) Numéro de dépôt international: FR9000007
(87) Numéro de publication internationale: WO9007949

(56) Documents cités:
- EP-A- 0 229 017
- WO-A-88/10127
- WO-A-89/06146
- DE-A- 1 965 761
- US-A- 3 934 586
- US-A- 3 951 146
- US-A- 4 731 068

## Description

La présente invention a pour objet des seringues jetables non réutilisables après une première injection et des procédés de fabrication de celles-ci.

On utilise, de plus en plus, des seringues jetables après usage pour éviter les risques de contamination en cas d'utilisation d'une seringue mal stérilisée après un premier usage.

Toutefois, de nombreuses contaminations sont dues à l'utilisation par des toxicomanes de seringues déjà utilisées et non stérilisées ou mal stérilisées.

Pour éviter de tels accidents on a proposé des seringues qui sont rendues automatiquement inutilisables après une première injection.

Le brevet US A 3 934 586 (Easton et AI) décrit des seringues pré-remplies qui permettent d'injecter plusieurs doses successives.

La tige de ces seringues porte des saillies qui viennent buter l'une après l'autre sur l'extrémité du réservoir et que l'on brise suivant une ligne oblique de moindre résistance qui laisse subsister une pointe qui raye la paroi du réservoir. De telles seringues ne peuvent être utilisées comme seringues vides permettant de prélever par aspiration une dose de liquide.

Les brevets DE A 1 965 761 - FR A 2 027 681 - US A 3 667 657 (Chiquiar-Arias) décrivent des seringues pré-remplies qui comportent un petit couteau qui est fixé latéralement sur la tige de manoeuvre et qui est engagé dans une ouverture du réservoir cylindrique. Ces seringues ne peuvent être vendues vides pour être remplies par l'utilisateur car dès que l'on enfonce le piston dans le réservoir le couteau découpe celui-ci.

Les brevets US A 3 951 146 (qui forme le préambule de la revendication 1) - DE A 2 354 628 et FR A 2 204 429 (Chiquiar-Arias) décrivent des seringues dans lesquelles la tige de manoeuvre du piston porte un boîtier ou une échancrure dans lequel est logé un petit couteau qui est poussé radialement par un ressort et qui présente un bord arrière arrondi et un bord avant tranchant. Ce document explique qu'il est possible de retirer le piston vers l'arrière pour aspirer du liquide dans le réservoir sans rayer la paroi du réservoir. Pour enfoncer le piston dans le fond du réservoir on engage une feuille de protection entre la paroi du réservoir cylindrique et la lame coupante que l'on retire ensuite.

Le problème à résoudre est de procurer des seringues auto-destructibles qui peuvent être vendues vides et qui permettent d'abord d'enfoncer le piston dans le réservoir pour chasser l'air sans rayer le réservoir puis de retirer le piston vers l'arrière pour aspirer un liquide sans rayer ni découper la paroi du réservoir puis d'enfoncer à nouveau le piston dans le réservoir pour injecter la dose de liquide contenue dans celui-ci en découpant ou en rayant ou en brisant la paroi du réservoir pour rendre la seringue inutilisable une deuxième fois.

Une seringue selon l'invention est du type connu comportant un réservoir cylindrique, un piston déplaçable à l'intérieur dudit réservoir, une tige de manoeuvre dudit piston et un organe coupant qui raye ou découpe ou brise la paroi dudit réservoir pendant l'injection du liquide contenu dans ledit réservoir.

La solution du problème posé consiste en une seringue dans laquelle une extrémité de la tige de manoeuvre et ledit piston sont reliés par des moyens qui permettent un déplacement relatif axial et l'un des deux porte au moins un doigt flexible, qui porte à son extrémité libre un organe coupant tandis que l'autre porte un moyen de déviation qui coopère avec ledit doigt flexible pour déformer celui-ci en repoussant vers l'extérieur ledit organe coupant lorsque ladite extrémité de la tige de manoeuvre et ledit piston se rapprochent l'un de l'autre de sorte que lorsqu'on retire la tige de manoeuvre vers l'arrière pour remplir la seringue du liquide à injecter lesdits doigts flexibles occupent une position dans laquelle lesdits organes c'upants sont effacés à l'intérieur dudit réservoir et ne touchent pas la paroi de celui-ci et lorsqu'on enfonce ledit piston dans ledit réservoir en poussant sur la tige de manoeuvre pour injecter le liquide lesdits doigts flexibles s'écartent et lesdits organes coupants sont repoussés vers l'extérieur et découpent ou rayent la paroi du réservoir ce qui rend la seringue inutilisable une deuxième fois.

Selon un premier mode de réalisation ledit piston ou une extrémité de la tige de manoeuvre porte une ou plusieurs rampes obliques par rapport à l'axe de la seringue contre lesquelles glissent lesdits doigts flexibles en s'écartant vers l'extérieur lorsque la tige de manoeuvre et ledit piston se rapprochent axialement l'un de l'autre et en s'effaçant vers l'intérieur lorsque la tige de manoeuvre et ledit piston s'éloignent axialement l'un de l'autre.

Selon un autre mode de réalisation chacun des doigts flexibles est relié au piston ou à une extrémité de ladite tige de manoeuvre par une biellette articulée qui repousse ledit doigt flexible vers l'extérieur lorsque ledit piston et ladite tige de manoeuvre se rapprochent axialement l'un de l'autre et qui ramène ledit doigt flexible vers l'intérieur lorsque ledit piston et ladite tige de manoeuvre s'éloignent axialement l'un de l'autre.

Un procédé de fabrication d'une seringue non réutilisable selon l'invention comporte les opérations suivantes :

- on relie une extrémité de la tige de manoeuvre audit piston par des moyens de liaison qui permettent un déplacement relatif axial de faible amplitude et on équipe respectivement l'un d'eux d'au moins un doigt flexible qui porte un organe coupant et l'autre d'un moyen de déviation qui coopère avec ledit doigt flexible pour écarter celui-ci vers l'extérieur lorsque ladite tige de manoeuvre et ledit piston se rapprochent axialement et pour permettre à celui-ci de s'effacer vers l'intérieur lorsque ledit piston et ladite tige de manoeuvre s'éloignent axialement l'un de l'autre.
- on place entre la collerette du réservoir et le disque de l'extrémité de la tige de manoeuvre une entretoise de sécurité afin d'éviter en cas d'appui accidentel sur la tige de manoeuvre, pendant la manutention, l'emballage ou le déballage de la seringue, que cet appui n'ait pour effet de percer avant utilisation la paroi du réservoir, rendant la seringue inutilisable prématurément.
- on connecte l'extrémité desdites seringues comportant le support d'aiguille ou l'aiguille incorporée à un moyen d'aspiration pour enfoncer ledit piston dans ledit réservoir sans que lesdits organes coupants ne découpent ou ne rayent ledit réservoir.

L'invention a pour résultat de nouvelles seringues jetables qui ne peuvent pas être réutilisées après une première injection.

Les seringues selon l'invention présentent l'avantage de pouvoir être livrées non remplies avec le piston enfoncé dans le réservoir de la seringue, cette opération étant réalisée en usine en connectant les seringues sur un moyen d'aspiration qui aspire le piston vers le fond de la seringue de telle sorte que les organes coupants sont effacés pendant cette opération et ne risquent pas de rayer ou de découper la paroi du réservoir.

Les seringues selon l'invention présentent également l'avantage que les organes coupants sont repoussés radialement par une force qui est proportionnelle à la poussée axiale exercée sur la tige de manoeuvre pendant l'injection de sorte que la pénétration des organes coupants dans la paroi du réservoir est mieux assurée que dans les seringues connues où le couteau est repoussé radialement par un ressort exerçant une poussée constante.

La liberté de déplacement relatif axial de la tige de manoeuvre par rapport au piston de la seringue permet d'obtenir un écartement des couteaux vers l'extérieur lorsqu'on appuie sur la tige de manoeuvre mais également un effacement des couteaux vers l'intérieur lorsque la tige de manoeuvre s'éloigne axialement du piston c'est-à-dire lorsqu'on tire sur la tige de manoeuvre pour aspirer du liquide ou bien lorsqu'on aspire le piston pour l'enfoncer dans la seringue de sorte qu'au cours de ces deux opérations les organes coupants ne risquent pas de découper ou de rayer la paroi du réservoir.

La description suivante se réfère aux dessins annexés qui représentent, sans aucun caractère limitatif, des exemples de réalisation de seringues selon l'invention.

La figure 1 représente en coupe axiale une seringue jetable selon un premier mode de réalisation.

La figure 2 représente en coupe axiale partielle la même seringue jetable selon l'invention en position de découpe.

La figure 3 est une coupe transversale selon la ligne III III de la figure 2.

La figure 4 représente en coupe axiale un deuxième mode de réalisation de seringues jetables selon l'invention.

La figure 5 représente, en coupe axiale partielle la même variante de réalisation que sur la figure 4, dans des conditions d'utilisations identiques à celles de la figure 2.

La figure 6 représente en coupe axiale un troisième mode de réalisation de seringues jetables selon l'invention.

La figure 7 représente en coupe axiale partielle la même seringue jetable en position de découpe.

La figure 8 représente en coupe axiale partielle un quatrième mode de réalisation de seringues jetables selon l'invention.

Les figures 9 et 10 représentent la mise en oeuvre du procédé de fabrication selon l'invention.

Les figures 11, 12 et 13 représentent trois modes de réalisation particuliers du réservoir cylindrique de seringues jetables selon l'invention.

La figure 14 est une coupe selon XIV XIV de la figure 11.

La figure 15 est une coupe selon XV XV de la figure 12.

La figure 16 est une coupe selon XVI XVI de la figure 13.

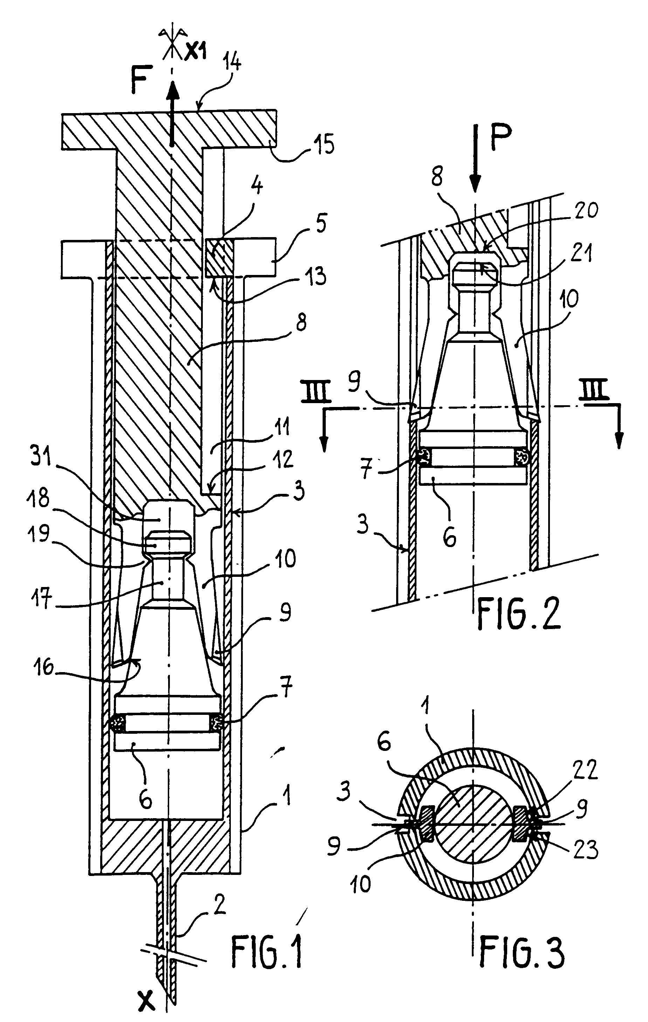

Comme indiqué dans les figures 1 à 3, une seringue jetable comporte de façon connue un réservoir cylindrique 1 d'axe XX1 qui est muni à une extrémité d'un dispositif destiné à recevoir une aiguille 2 ou d'une aiguille incorporée, une tige de manoeuvre 8 et un piston 6 qui coulissent dans ledit réservoir.

Ladite tige de manoeuvre comporte typiquement une rainure 11, longitudinale et parallèle à l'axe, ladite rainure étant limitée à une extrémité par un disque 15 et à l'autre extrémité par une butée 12 liée à ladite tige de manoeuvre.

Ledit réservoir comporte avantageusement à une extrémité une collerette 5 formant bride sur laquelle est rapporté, après mise en place de ladite tige de manoeuvre dans ledit réservoir, un talon de guidage 4 qui pénètre dans ladite rainure.

Le jeu existant entre ledit talon et ladite rainure autorise le coulissement de ladite tige de manoeuvre par rapport audit réservoir mais interdit la rotation de ladite tige par rapport audit réservoir.

Ledit talon 4 coopère par sa face 13 qui forme butée, avec la butée 12 liée à la tige de manoeuvre, de telle sorte que lorsqu'on tente d'extraire ladite tige dudit réservoir, la butée 12 vient en contact avec la butée 13 et interdit donc l'extraction.

Cette interdiction évite que l'utilisateur n'enlève les organes coupants.

Dans un mode de réalisation préférentiel, la paroi dudit réservoir comporte au moins une zone 3 de moindre résistance, par exemple une rainure qui s'étend selon une génératrice dudit réservoir cylindrique sensiblement sur toute la hauteur dudit réservoir.

Avantageusement ledit piston comporte des moyens d'étanchéité 7.

La seringue selon l'invention comporte au moins une saillie appointée et tranchante ou tout autre moyen 9 contondant ou coupant ou tranchant ou brisant tel que lame, pointe ou molette 30 apte à réaliser le rainurage, la déformation notable et permanente, la découpe, la perforation ou la rupture fragmentaire dudit réservoir de telle sorte que celui-ci ne puisse plus coopérer avec ledit piston de manière à provoquer le pompage du fluide, et donc empêche l'utilisation ultérieure de la seringue aux fins d'injection dudit fluide.

Dans la suite du document, afin d'en faciliter la lecture, lesdits moyens seront désignés de façon générique par le terme d'organe coupant 9.

Dans les modes de réalisation selon l'invention, lesdits organes coupants sont montés à l'extrémité libre de doigts flexibles 10 qui sont eux-mêmes liés à l'un ou l'autre d'une extrémité de ladite tige de manoeuvre ou dudit piston.

Dans le mode de réalisation représenté figure 3, la seringue comporte deux doigts flexibles.

Dans les modes de réalisation selon l'invention représentés sur les figures 1, 2, 4, 5, 6, 7, 8, 9, lesdits doigts flexibles sont liés à une extrémité de ladite tige de manoeuvre.

Dans un premier mode de réalisation selon l'invention, ledit piston comporte au moins une surface de déviation 16 qui est constituée par exemple par une surface tronconique comme représentée sur les figures 1 et 2, ou bien par une rampe oblique par rapport à l'axe XX₁, contre laquelle les doigts viennent au contact.

Sur la figure 1, ladite surface de déviation coopère avec lesdits doigts flexibles qui occupent une position dans laquelle ceux-ci sont effacés à l'intérieur dudit réservoir et ne touchent pas la paroi de celui-ci.

Sur la figure 2 ladite surface de déviation coopère avec lesdits doigts flexibles pour déformer ceux-ci en repoussant vers l'extérieur lesdits organes coupants lorsque ladite extrémité de la tige de manoeuvre et ledit piston se rapprochent l'un de l'autre, consécutivement à l'application d'une poussée représentée par la flèche P sur ladite tige de manoeuvre de telle sorte que lesdits organes coupants découpent ou rayent la paroi dudit réservoir, ce qui rend la seringue inutilisable une deuxième fois.
Ces changements de position desdits doigts flexibles sont dus d'une part à la flexibilité des doigts et d'autre part au rapprochement ou à l'éloignement relatif dudit piston et de ladite tige de manoeuvre, mouvements rendus possibles par la structure particulière des moyens de liaison entre ladite tige de manoeuvre et ledit piston.

En effet, dans ce mode de réalisation, les figures 1 et 2 montrent que ledit piston comporte une tige 17 et un renflement 18 qui se termine par une face 21, qu'une extrémité de ladite tige de manoeuvre comporte un logement 31 limité par un fond 20, et que lesdits doigts flexibles comportent des ergots 19.

La flexibilité des doigts permet lors du montage de la seringue de laisser pénétrer ledit renflement du piston dans ledit logement de la tige de manoeuvre jusqu'à ce que lesdits ergots s'engagent derrière ledit renflement et relie ainsi ladite tige de manoeuvre et ledit piston de telle sorte que ceux-ci conservent une liberté de déplacement relatif axial.

La figure 2 montre que ledit déplacement relatif axial est limité dans le sens du rapprochement entre ladite tige et ledit piston par le contact de ladite surface 21 et dudit fond 20, de telle sorte que l'écartement des doigts flexibles consécutif à ce rapprochement est ainsi limité.

De plus, la figure 3 montre que lesdits doigts flexibles comportent des faces d'appui 22 et 23 situés à proximité immédiate des organes coupants 9, lesdites faces d'appui coopérant avec la paroi intérieure dudit réservoir, limitant ainsi l'écartement desdits organes coupants, de telle sorte que pendant la découpe les organes coupants ne font pas saillie à l'extérieur dudit réservoir et ne peuvent pas blesser l'utilisateur.

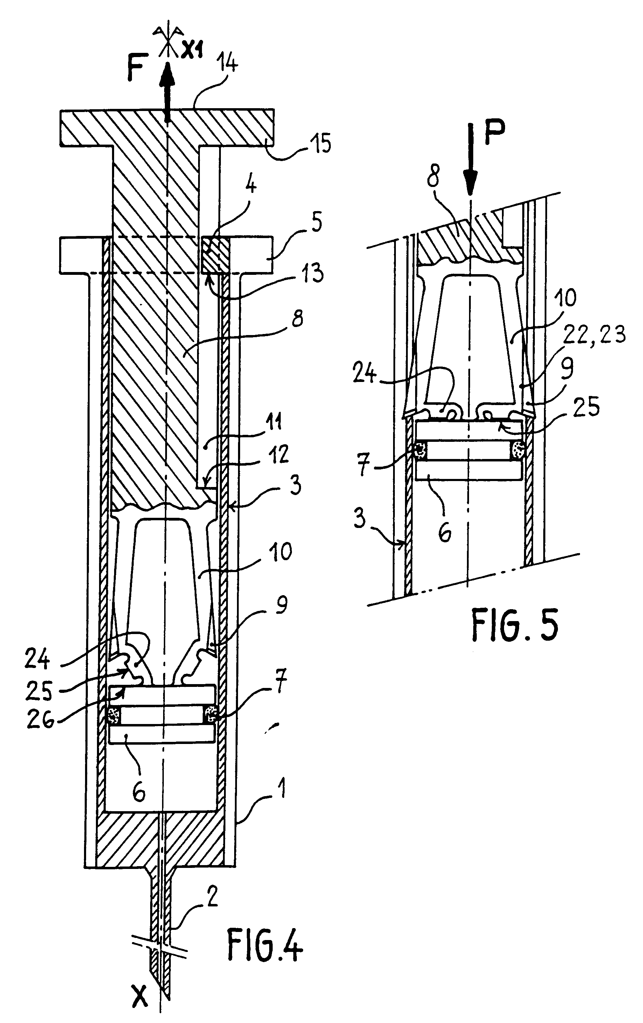

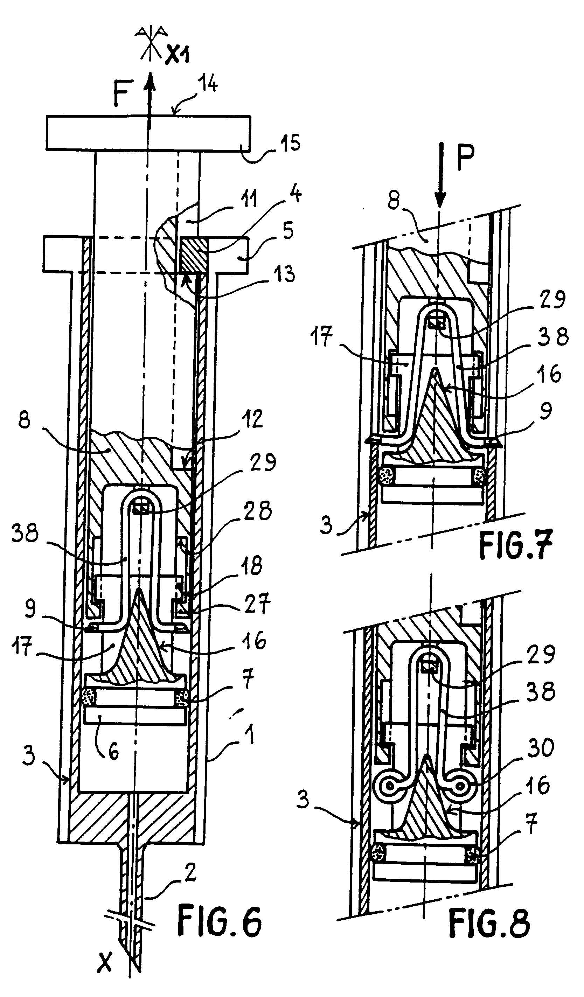

Sur les figures 4 et 5 les parties homologues des figures 1 et 2 sont représentées par les mêmes repères.

Les figures 4 et 5 montrent un autre mode de réalisation selon l'invention dans lequel lesdits doigts flexibles 10 sont reliés par une extrémité à ladite tige de manoeuvre et par l'autre extrémité audit piston 6 par l'intermédiaire de biellettes 24 articulées à leurs deux extrémités, les moyens de liaison entre ladite tige de manoeuvre et ledit piston ainsi définis permettant l'écartement et l'effacement desdits organes coupants correspondant respectivement au rapprochement et à l'éloignement relatif de ladite tige de manoeuvre et dudit piston.

En effet, les figures 4 et 5 montrent que lesdites biellettes comportent une partie centrale sensiblement rigide qui elle-même comporte une surface 25 qui forme butée, et deux extrémités de section et de rigidité sensiblement moindre que la partie centrale, de telle sorte que lesdites extrémités de la biellette qui sont liées, pour l'une audit piston, pour l'autre audit doigt flexible, peuvent se déformer et constituer ainsi une articulation.

Dans la figure 4, on voit que, consécutivement à l'application d'une traction sur la tige de manoeuvre représentée par la flèche F, les forces de traction sont transmises par lesdits doigts flexibles auxdites biellettes et par lesdites biellettes audit piston.

Compte tenu de la résistance au coulissement dudit piston dans ledit réservoir dû au frottement de celui-ci et desdits moyens d'étanchéité, les extrémités desdites biellettes jouant le rôle d'articulation en se déformant, lesdites biellettes se déplacent en provoquant l'effacement à l'intérieur du réservoir des organes coupants simultanément à l'éloignement relatif de ladite tige de manoeuvre et dudit piston.

La figure 5 montre que, consécutivement à l'application d'une poussée sur la tige de manoeuvre, représentée par la flèche P, les efforts transmis par lesdits doigts flexibles conduisent à une déformation des extrémités des biellettes 24 jusqu'à ce que l'une au moins des butées 22, 23 et 25 produise ses effets ; cette déformation des extrémités des bielles provoque la déviation vers la paroi dudit réservoir des organes coupants 9, de telle sorte que, lors de l'enfoncement du piston dans ledit réservoir, lesdits organes coupants découpent ou rayent ladite paroi intérieure dudit réservoir.

Les figures 6 et 7 représentent un troisième mode de réalisation selon l'invention. Les parties homologues des figures 1 et 2 sont représentées par les mêmes repères.

Le piston 6 comporte une tige 17 et un renflement 18 qui peut coulisser dans un logement 31 aménagé à l'extrémité de ladite tige de manoeuvre 8 qui elle-même comporte des ergots 27 et une face d'appui 28 ce qui permet un déplacement relatif axial entre la tige de manoeuvre et le piston.

A l'intérieur de ladite tige de piston 17 sont construites au moins deux surfaces de déviation 16 qui forment deux rampes obliques qui convergent l'une vers l'autre dans la direction de la tige de manoeuvre.

Une épingle élastique 38 en forme de U constitue une paire de doigts flexibles dont les extrémités sont coudées et portent les organes coupants 9.

Ladite épingle est maintenue solidaire de ladite tige de manoeuvre par un moyen de liaison 29 tel que pince, anneau ou tout autre moyen de fixation équivalent.

Sur la figure 6, la surface de déviation 16 coopère avec lesdits doigts flexibles de telle sorte que ceux-ci s'effacent à l'intérieur dudit réservoir cylindrique sans toucher la paroi de celui-ci, lorsque ledit piston s'éloigne de ladite tige de manoeuvre.

La figure 7 montre que pendant l'injection du liquide sous l'action d'une poussée de l'utilisateur sur ladite tige de manoeuvre, représentée par la flèche P, ladite surface de déviation repousse vers l'extérieur, du fait du rapprochement dudit piston et de ladite tige de manoeuvre, lesdits organes coupants qui découpent ou rayent ladite paroi.

Avantageusement, dans le mode de réalisation représenté sur les figures 6 et 7, les surfaces de déviation, sous formes de rampes inclinées, peuvent être constituées par le fond d'une rainure aménagée dans ladite tige 17, les flancs de ladite rainure servant à guider les doigts flexibles lors des mouvements relatifs entre ledit piston et ladite tige de manoeuvre.

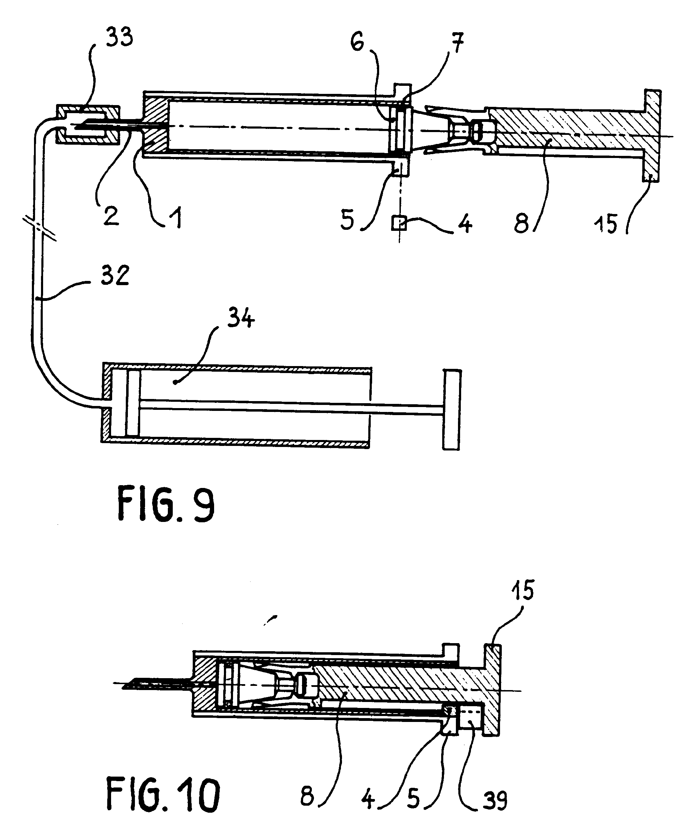

La figure 8 montre une variante de réalisation selon l'invention, dans laquelle les organes coupants sont constitués par des molettes 30, notamment pour le cas ou ledit réservoir est fabriqué dans un matériau cassant tel que le verre, les matières plastiques de type polycarbonate, qui, sous la pression exercée par lesdites molettes selon les mêmes principes que développé pour les autre réalisations, subira une rupture fragmentaire ou éclatement.

Dans ce mode de réalisation, le réservoir cylindrique peut être recouvert extérieurement d'une pellicule souple qui forme une chemise évitant la dispersion des fragments résultant de la rupture. Avantageusement, la rupture se produit dans la zone de moindre résistance 3 constituée par une rainure réalisée dans la masse, ladite rainure étant remplie d'une résine souple qui évite la dispersion des fragments résultant de la rupture.

La figure 9 représente les moyens mis en oeuvre dans le procédé de fabrication des seringues jetables selon l'invention, et plus particulièrement dans le procédé qui consiste à amener ledit piston dans le fond dudit réservoir sans détériorer celui-ci et permettre de livrer des seringues vides et prêtes à être remplies par aspiration.

Les moyens comportent au moins un tuyau 32, équipé d'un dispositif de connexion 33, lequel dispositif peut être fixé sur l'extrémité de la seringue destinée à recevoir les aiguilles, ou directement à l'extrémité de l'aiguille incorporée.

Ledit tuyau est relié à des moyens d'aspiration 34 tels qu'une pompe par exemple.

Les quatres principales pièces composant la seringue soit ledit réservoir, ledit piston, ladite tige de manoeuvre, ledit talon, peuvent être fabriqués séparément.

Dans ce cas, on engage ledit renflement dudit piston dans ledit logement prévu dans ladite tige de manoeuvre.

En variante la tige et le piston sont solidaires.

On présente ensuite ledit piston dans ledit réservoir de telle sorte que les moyens d'étanchéité 7 soient introduits comme représenté sur la figure 9 en ayant soin de maintenir ladite tige de manoeuvre éloignée dudit piston grâce à la structure des moyens de liaison décrits dans les paragraphes ci-dessus, de telle sorte que lesdits doigts flexibles soient dans une position effacée et ne détériorent pas ledit réservoir lorsqu'on les fait pénétrer dans celui-ci.

Lorsqu'on actionne ledit moyen d'aspiration, ledit piston est soumis à une force due à la différence de pression existant entre ses faces, laquelle force provoque son déplacement vers le fond dudit réservoir ; les doigts flexibles restent dans leur position effacée durant ce mouvement. Le piston entraîne la tige de manoeuvre dans son mouvement. On monte enfin ledit talon 4 sur ladite collerette et l'on place entre la collerette 5 du réservoir et le disque 15 de l'extrémité de la tige de manoeuvre comme le montre la figure 10, une entretoise de sécurité 39 afin d'éviter en cas d'appui accidentel sur la tige de manoeuvre, pendant la manutention, l'emballage ou le déballage de la seringue, que cet appui n'ait pour effet de découper ou de rayer avant utilisation la paroi du réservoir, rendant la seringue inutilisable prématurément.

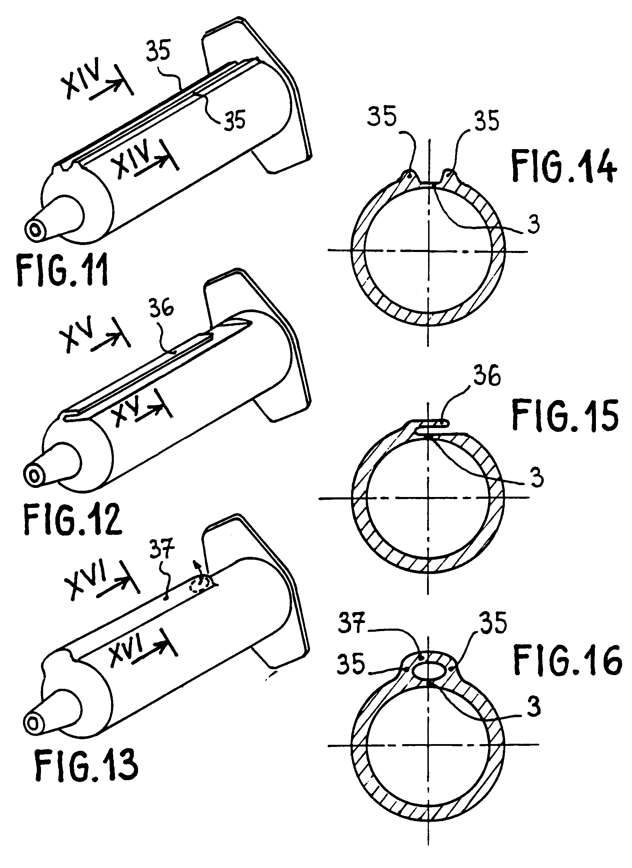

Les figures 11 à 16 représentent trois autres modes de réalisation d'une seringue selon l'invention dans lequel ledit réservoir comporte ladite zone de moindre résistance 3 qui s'étend selon une génératrice dudit réservoir cylindrique.

Dans un mode de réalisation représenté sur les figures 11 et 14, ladite paroi dudit réservoir comporte deux zones proéminentes 35 qui s'étendent selon deux génératrices dudit réservoir, de part et d'autre de ladite zone de moindre résistance 3, et forment deux bourrelets de telle sorte que lors de la découpe dudit réservoir par lesdits organes coupants, lesdites zones proéminentes interdisent tout contact accidentel entre lesdits organes coupants et l'utilisateur.

Les figures 12 et 15 représentent une autre variante dans laquelle ladite paroi comporte une seule zone proéminente, en forme de languette 36, qui recouvre partiellement ladite zone de moindre résistance 3.

Les figures 13 et 16 représentent une variante selon l'invention dans laquelle lesdites zones proéminentes 35 situées de part et d'autre de ladite zone 3, se rejoignent pour former une languette 37 qui recouvre entièrement ladite zone 3.

Les modes de réalisation d'une seringue selon l'invention représentés sur les figures 11 à 16 permettent d'éviter tout risque de blessure de l'utilisateur par lesdits organes coupants.

Bien entendu les dispositions relatives des doigts et des surfaces de déviations par rapport au piston et à la tige pourraient être inversées sans que le fonctionnement soit modifié.

## Revendications

1. Seringue jetable et non réutilisable après une première injection comportant un réservoir cylindrique (1), un piston (6) déplaçable à l'intérieur dudit réservoir, une tige de manoeuvre (8) dudit piston et un organe coupant (9) qui raye ou découpe la paroi dudit réservoir pendant l'injection du liquide contenu dans ledit réservoir caractérisée en ce qu'une extrémité de ladite tige de manoeuvre et ledit piston sont reliés par des moyens qui permettent un déplacement relatif axial et l'un des deux porte au moins un doigt flexible (10), qui porte à son extrémité libre un organe coupant (9) tandis que l'autre porte un moyen de déviation (16) qui coopère avec ledit doigt flexible pour déformer celui-ci en repoussant vers l'extérieur ledit organe coupant lorsque ladite extrémité de la tige de manoeuvre et ledit piston se rapprochent l'un de l'autre de sorte que lorsqu'on enfonce ledit piston dans ledit réservoir en poussant sur la tige de manoeuvre lesdits doigts flexibles s'écartent et lesdits organes coupants découpent ou rayent la paroi du réservoir ce qui rend la seringue inutilisable une deuxième fois et lorsqu'on retire la tige de manoeuvre vers l'arrière lesdits doigts flexibles prennent une position dans laquelle lesdits organes coupants sont effacés à l'intérieur dudit réservoir et ne touchent pas la paroi de celui-ci.

2. Seringue selon la revendication 1 caractérisée en ce que ledit piston ou une extrémité de ladite tige de manoeuvre porte une ou plusieurs rampes obliques (16) par rapport à l'axe de la seringue contre lesquelles glissent lesdits doigts flexibles en s'écartant vers l'extérieur lorsque la tige de manoeuvre et ledit piston se rapprochent axialement l'un de l'autre et en s'effaçant vers l'intérieur lorsque la tige de manoeuvre et ledit piston s'éloignent axialement l'un de l'autre.

3. Seringue selon la revendication 1 caractérisée en ce que l'un ou l'autre dudit piston ou d'une extrémité de ladite tige de manoeuvre porte une surface conique centrée sur l'axe de la seringue contre laquelle glissent lesdits doigts flexibles en s'écartant vers l'extérieur ou en s'effaçant vers l'intérieur selon que ledit piston et ladite extrémité de la tige de manoeuvre se rapprochent ou s'éloignent axialement l'un de l'autre.

4. Seringue selon l'une quelconque des revendications 1 à 3 caractérisée en ce que ledit piston est prolongé par une tige (17) portant à son extrémité libre un renflement (18) et ladite tige de manoeuvre comporte des ergots (19, 27) lesquels ergots s'engagent derrière ledit renflement en reliant ladite tige de manoeuvre et ledit piston de telle sorte qu'ils conservent une liberté de déplacement relatif axial de faible amplitude.

5. Seringue selon la revendication 1 caractérisée en ce que chacun desdits doigts flexibles est relié audit piston ou à une extrémité de ladite tige de manoeuvre par une biellette (24) articulée qui repousse ledit doigt flexible vers l'extérieur lorsque ledit piston et ladite tige de manoeuvre se rapprochent axialement l'un de l'autre et qui ramène ledit doigt flexible vers l'intérieur lorsque ledit piston et ladite tige de manoeuvre s'éloignent axialement l'un de l'autre.

6. Seringue selon la revendication 1 dans laquelle ledit réservoir est constitué d'un matériau cassant tel que le verre ou le polycarbonate caractérisée en ce que lesdits organes coupants sont des molettes (30) dont la pression contre la paroi intérieure provoque la rupture fragmentaire dudit réservoir le long de zones de moindre résistance.

7. Seringue selon la revendication 1 caractérisée en ce que lesdits organes coupants sont portés par une épingle élastique (38) qui constitue lesdits doigts flexibles et qui est fixée à ladite tige de manoeuvre.

8. Seringue selon les revendications 1 à 7 dans laquelle ledit réservoir cylindrique comporte au moins une zone de moindre résistance (3) le long d'une génératrice caractérisée en ce que ladite zone (3) est bordée par deux bourrelets (35) faisant saillie par rapport à la paroi externe dudit réservoir.

9. Seringue selon les revendications 1 à 7 dans laquelle ledit réservoir cylindrique comporte au moins une zone de moindre résistance (3) le long d'une génératrice caractérisée en ce que ladite zone (3) est recouverte au moins en partie par une languette (36), (37).

10. Procédé de fabrication d'une seringue jetable et non réutilisable après une première injection du type comportant un réservoir cylindrique, un piston déplaçable à l'intérieur dudit réservoir, une tige de manoeuvre dudit piston et des moyens coupants qui rayent ou découpent la paroi dudit réservoir pendant l'injection du liquide contenu dans ledit réservoir caractérisé en ce qu'on relie une extrémité de la tige de manoeuvre audit piston par des moyens de liaison qui permettent un déplacement relatif axial de faible amplitude et on équipe respectivement l'un d'eux d'au moins un doigt flexible qui porte un organe coupant et l'autre d'un moyen de déviation qui coopère avec ledit doigt flexible pour écarter celui-ci vers l'extérieur lorsque ladite tige de manoeuvre et ledit piston se rapprochent axialement et pour permettre à celui-ci de s'effacer vers l'intérieur lorsque ledit piston et ladite tige de manoeuvre s'éloignent axialement l'un de l'autre.

11. Procédé selon la revendication 10 de fabrication d'une seringue comportant un support d'aiguille ou une aiguille incorporée caractérisé en ce que l'on connecte l'extrémité desdites seringues comportant ledit support d'aiguille ou ladite aiguille incorporée à un moyen d'aspiration (34) pour enfoncer ledit piston dans ledit réservoir sans que lesdits organes coupants ne découpent ou ne rayent ledit réservoir.

12. Procédé selon l'une quelconque des revendications 10 à 11 caractérisé en ce que l'on place entre la collerette (5) du réservoir et le disque (15) de l'extrémité de la tige de manoeuvre une entretoise de sécurité (39) pour éviter,en cas d'appui accidentel sur la tige de manoeuvre, pendant la manutention, l'emballage ou le déballage de la seringue, que ledit appui accidentel n'ait pour effet de découper ou de rayer avant utilisation la paroi du réservoir, rendant la seringue inutilisable prématurément.

## Patentansprüche

1. Wegwerfspritze zur einmaligen Verwendung, umfassend eine zylindrische Kammer (1), einen innerhalb der besagten Kammer verschiebbaren Kolben (6), einen Betätigungsschaft (8) des besagten Kolbens und eine Schnittvorrichtung (9), die die Wand der besagten Kammer während des Einspritzens der in dieser Kammer enthaltenen Flüssigkeit einritzt oder zerschneidet, dadurch gekennzeichnet, daß ein Ende des besagten Betätigungsschafts und der Kolben durch Vorrichtungen miteinander verbunden sind, die ihre jeweilige Axialbewegung zulassen, wobei einer von beiden mindestens einen beweglichen Finger (10) aufweist, der an seinem freien Ende eine Schnittvorrichtung (9) aufweist, während der andere eine Ablenkvorrichtung (16) aufweist, die mit dem genannten beweglichen Finger zusammen funktioniert, um diesen zu verformen, indem sie die genannte Schnittvorrichtung nach außen drückt, wenn das genannte Ende des Betätigungsschafts und der Kolben aufeinander zulaufen, so daß, wenn man den genannten Kolben in die besagte Kammer drückt, indem man auf den Betätigungsschaft drückt, sich die genannten beweglichen Finger spreizen und die genannten Schnittvorrichtungen die Wand der Kammer zerschneiden oder einritzen, wodurch eine Wiederverwendung der Spritze unmöglich gemacht wird, und, wenn man den Betätigungsschaft zurückzieht, die besagten beweglichen Finger eine Lage einnehmen, in der die besagten Schnittvorrichtungen in das Innere der Kammer eingezogen werden und deren Wand nicht berühren.

2. Spritze nach Anspruch 1, dadurch gekennzeichnet, daß der Kolben oder ein Ende des Betätigungsschafts eine oder mehrere bezüglich der Achse der Spritze abgeschrägte Stellen (16) aufweist, gegen die die genannten beweglichen Finger gleiten, wenn sie sich nach außen spreizen, wenn sich Betätigungsschaft und Kolben axial einander annähern, und wenn sie nach innen zurückgehen, wenn der Betätigungsschaft und der Kolben sich axial voneinander entfernen.

3. Spritze nach Anspruch 1, dadurch gekennzeichnet, daß entweder der Kolben oder ein Ende des besagten Betätigungsschafts eine konische Fläche aufweisen, die auf die Achse der Spritze zentriert ist, gegen die die genannten beweglichen Finger gleiten, wenn sie sich nach außen spreizen oder nach innen zurückweichen, je nach dem, ob sich der Kolben oder das Ende des Betätigungsschafts einander axial annähern oder voneinander entfernen.

4. Spritze nach einem der Ansprüche 1 bis 3, dadurch gekennzeichnet, daß der Kolben durch einen Schaft (17) verlängert ist, der an seinem freien Ende eine Verstärkung (18) aufweist, und daß der Betätigungsschaft Nocken (19, 27) aufweist, die hinter diese Verstärkung fassen und dabei den Betätigungsschaft und den Kolben so verbinden, daß beide eine geringe axiale Bewegungsfreiheit zueinander behalten.

5. Spritze nach Anspruch 1, dadurch gekennzeichnet, daß jeder der beweglichen Finger mit dem Kolben oder einem Ende des Betätigungsschafts über ein schwenkbares Zwischenglied (24) verbunden ist, das den besagten beweglichen Finger nach außen drückt, wenn der Kolben und der Betätigungsschaft einander axial annähern, und das den beweglichen Finger nach innen einzieht, wenn der Kolben und der Betätigungsschaft sich axial voneinander entfernen.

6. Spritze nach Anspruch 1, bei der die besagte Kammer aus zerbrechlichem Material wie Glas oder Polycarbonat besteht, dadurch gekennzeichnet, daß die Schnittvorrichtungen aus einem Rädchen (30) bestehen, durch dessen Drücken gegen die Innenwand das Brechen der Kammer entlang von Sollbruchstellen erreicht wird.

7. Spritze nach Anspruch 1, dadurch gekennzeichnet, daß die Schnittvorrichtungen an einer elastischen Nadel (38) sitzen, die die besagten beweglichen Finger bildet und am Betätigungsschaft befestigt ist.

8. Spritze nach den Ansprüchen 1 bis 7, bei der die zylindrische Kammer entlang einer Erzeugenden mindestens eine Sollbruchstelle (3) aufweist, dadurch gekennzeichnet, daß diese Sollbruchstelle (3) von zwei Verdickungen (35) gesäumt ist, die gegenüber der Außenwand der besagten Kammer überstehen.

9. Spritze nach den Ansprüchen 1 bis 7, bei der die besagte zylindrische Kammer entlang einer Erzeugenden mindestens eine Sollbruchstelle (3) aufweist, dadurch gekennzeichnet, daß die genannte Stelle (3) zumindest teilweise von einer Zunge (36), (37) bedeckt ist.

10. Herstellungsverfahren für eine Wegwerfspritze vom Typ umfassend eine zylindrische Kammer, einen innerhalb der besagten Kammer verschiebbaren Kolben, einen Betätigungsschaft für den besagten Kolben und eine Schnittvorrichtung, die die Wand der besagten Kammer während des Einspritzens der in dieser Kammer enthaltenen Flüssigkeit einritzt oder zerschneidet, dadurch gekennzeichnet, daß ein Ende des besagten Betätigungsschafts und der Kolben durch Vorrichtungen miteinander verbunden werden, die ihre Axialbewegung zueinander in einem geringen Maß zulassen, wobei man jeweils einen von beiden mit mindestens einem beweglichen Finger versieht, der an seinem freien Ende eine Schnittvorrichtung aufweist, während der andere eine Ablenkvorrichtung aufweist, die mit dem genannten beweglichen Finger zusammen funktioniert, um diesen nach außen zu spreizen, wenn der Betätigungsschaft und der Kolben einander axial nähern, und diesem zu ermöglichen, nach innen zu weichen, wenn sich der Kolben und der Betätigungsschaft axial voneinander entfernen.

11. Verfahren nach Anspruch 10 zur Herstellung einer Spritze umfassend eine Nadelhalterung oder eine eingearbeitete Nadel, dadurch gekennzeichnet, daß man das Endstück der besagten Spritzen, das die besagte Nadelhalterung oder eingearbeitete Nadel aufweist, mit einer Ansaugvorrichtung (34) verbindet, um den Kolben in die besagte Kammer zu drücken, ohne daß die genannten Schnittvorrichtungen die Kammer einritzen oder zerschneiden.

12. Verfahren nach einem der Ansprüche 10 bis 11, dadurch gekennzeichnet, daß man zwischen dem Kragen (5) der Kammer und der Scheibe (15) am Ende des Betätigungsschafts eine Sicherheitssperre (39) vorsieht, damit im Falle eines versehentlichen Drückens auf den Betätigungsschaft während des Transports, Verpackens oder Auspackens dieses Drücken die Wand der Kammer nicht vor der Benutzung einritzt oder zerschneidet und die Spritze vorzeitig unbrauchbar macht.

## Claims

1. Disposable syringe non-reusable after a first injection comprising a cylindrical barrel (1), a piston (6) displaceable within said barrel, a rod (8) for manoeuvring said piston and a cutting member (9) which scratches or cuts the wall of said barrel during injection of the liquid contained in said barrel, characterized in that one end of said manoeuvring rod and said piston are connected by means which allow a relative axial displacement and one of the two bears at least one flexible finger (10) which bears at its free end a cutting member (9), whilst the other bears deviation means (16) which cooperates with said flexible finger in order to deform the latter by pushing outwardly said cutting member when said end of the manoeuvring rod and said piston approach each other so that, when said piston is driven into said barrel, pushing on the manoeuvring rod, said flexible fingers move apart and said cutting members cut or scratch the wall of the barrel, which renders the syringe unusable a second time and when the manoeuvring rod is withdrawn rearwardly, said flexible fingers take a position in which said cutting members are retracted inside said barrel and do not touch the wall thereof.

2. Syringe according to claim 1, characterized in that said piston or an end of said manoeuvring rod bears one or more oblique ramps (16) with respect to the axis of the syringe against which slide said flexible fingers, moving apart towards the outside when the manoeuvring rod and said piston approach each other axially and retracting inwardly when the manoeuvring rod and said piston move axially apart from each other.

3. Syringe according to claim 1, characterized in that one or the other of said piston or an end of said manoeuvring rod bears a conical surface centred on the axis of the syringe against which said flexible fingers slide, moving apart towards the outside or retracting inwardly, depending on whether said piston and said end of the manoeuvring rod approach or move axially apart from each other.

4. Syringe according to any one of claims 1 to 3, characterized in that said piston is extended by a rod (17) bearing at its free end a swelling (18) and said manoeuvring rod comprises catches (19, 27), said catches engage behind said swelling, connecting said manoeuvring rod and said piston so that they conserve a freedom of relative axial displacement of small amplitude.

5. Syringe according to claim 1, characterized in that each of said flexible fingers is connected to said piston or to an end of said manoeuvring rod by an articulated connecting rod (24) which pushes said flexible finger outwardly when said piston and said manoeuvring rod approach each other axially and which returns said flexible finger inwardly when said piston and said manoeuvring rod move axially away from each other.

6. Syringe according to claim 1 in which said barrel is constituted by a breakable material such as glass or polycarbonate, characterized in that said cutting members are knurls (30) of which the pressure against the inner wall provokes breakage of said barrel into fragments along zones of least resistance.

7. Syringe according to claim 1, characterized in that said cutting members are borne by an elastic pin (38) which constitutes said flexible fingers and which is fixed to said manoeuvring rod.

8. Syringe according to claims 1 to 7 in which said cylindrical barrel comprises at least one zone of least resistance (3) along a generatrix, characterized in that said zone (3) is bordered by two beads (35) projecting with respect to the outer wall of said barrel.

9. Syringe according to claims 1 to 7 in which said cylindrical barrel comprises at least one zone of least resistance (3) along a generatrix, characterized in that said zone (3) is covered at least partly by a tongue (36), (37).

10. Process for manufacturing a disposable syringe non-reusable after a first injection, of the type comprising a cylindrical barrel , a piston displaceable inside said barrel, a rod for manoeuvring said piston and cutting means which scratch or cut the wall of said barrel during injection of the liquid contained in said barrel, characterized in that one end of the manoeuvring rod is connected to said piston by connecting means which allow a relative axial displacement of small amplitude and one of them is respectively equipped with at least one flexible finger which bears a cutting member and the other with a deviation means which cooperates with said flexible finger to move the latter away outwardly when said manoeuvring rod and said piston approach axially, and to allow the latter to retract inwardly when said piston and said manoeuvring rod move axially away from each other.

11. Process according to claim 10 for manufacturing a syringe comprising a needle support or an incorporated needle, characterized in that the end of said syringes comprising said needle support or said incorporated needle is connected to a suction means (34) for driving said piston in said barrel without said cutting members cutting or scratching said barrel.

12. Process according to any one of claims 10 to 11, characterized in that there is placed between the flange (5) of the barrel and the disc (15) of the end of the manoeuvring rod a safety distance piece (39) in order to avoid, in the event of accidental abutment on the manoeuvring rod during handling, packing or unpacking of the syringe, that such accidental abutment cuts or scratches, before use, the wall of the barrel, prematurely rendering the syringe unusable.
